# FASCICULE DE BREVET EUROPEEN

(11) **EP 1 631 320 B1**
(45) Date de publication et mention de la délivrance du brevet: **06.12.2006**
(21) Numéro de dépôt: 04742597.0
(22) Date de dépôt: 28.04.2004
(51) Int. Cl.: A61K 51/08

(54) **UTILISATION D UN ANALOGUE DU TETRAPEPTIDE ACSDKP RESISTANT A L'ENZYME DE CONVERSION DE L'ANGIOTENSINE I, POUR LA MESURE DU DEBIT DE FILTRATION GLOMERULAIRE CHEZ L'HOMME OU L'ANIMAL ET KIT LE CONTENANT**
VERWENDUNG EINES TETRAPEPTID-ACSDKP-ANALOGONS MIT RESISTENZ GEGEN DAS ANGIOTENSIN I CONVERSION ENZYM, ZUR MESSUNG DER GLOMERULÄREN FILTRATIONSRATE IN EINEM MENSCHEN ODER TIER UND DIESES ENTHALTENDES KIT
USE OF A TETRAPEPTIDE ACSDKP ANALOGUE RESISTANT TO THE ANGIOTENSIN I CONVERSION ENZYME, FOR MEASURING GLOMERULAR FILTRATION RATE IN A HUMAN OR AN ANIMAL AND KIT CONTAINING SAME

(30) Priorité: 29.04.2003 FR 0305228
(43) Date de publication de la demande: 08.03.2006
(73) Titulaire: ASSISTANCE PUBLIQUE, HOPITAUX DE PARIS, 75004 Paris (FR); COMMISSARIAT A L'ENERGIE ATOMIQUE, 75015 Paris (FR)
(72) Inventeur: AZIZI, Michel, F-75015 Paris (FR); EZAN, Eric, F-92240 Malakoff (FR)
(74) Mandataire: Vialle-Presles, Marie José
(86) Numéro de dépôt international: PCT/FR2004/001031
(87) Numéro de publication internationale: WO 2004/096292

(56) Documents cités:
- JUNOT CHRISTOPHE ET AL: "Development of an enzyme immunoassay for a stable amidated analog of the hemoregulatory peptide Acetyl-Ser-Asp-Lys-Pro" JOURNAL OF IMMUNOASSAY AND IMMUNOCHEMISTRY, vol. 22, no. 1, février 2001 (2001-02), pages 15-31, XP009023935 ISSN: 1532-1819
- GAUDRON S ET AL: "NAcSDKP Analogs Resistant to Angiotensin-Converting Enzyme" JOURNAL OF MEDICINAL CHEMISTRY, AMERICAN CHEMICAL SOCIETY. WASHINGTON, US, vol. 40, 1997, pages 3963-3968, XP002223609 ISSN: 0022-2623
- AZIZI MICHEL ET AL: "Renal and metabolic clearance of N-acetyl-seryl-aspartyl-lysyl-proline (AcSDKP) during angiotensin-converting enzyme inhibition in humans" HYPERTENSION (BALTIMORE), vol. 33, no. 3, mars 1999 (1999-03), pages 879-886, XP002267005 ISSN: 0194-911X

## Description

La présente invention est relative à la mesure du débit de filtration glomérulaire (DFG) chez l'homme normal ou malade ainsi que chez l'animal, par une nouvelle méthode mettant en oeuvre un analogue du tétrapeptide N-acétyl-Ser-Asp-Lys-Pro (AcSDKP), résistant à l'enzyme de conversion de l'angiotensine I. La mesure du DFG permet d'évaluer avec précision la fonction rénale ; elle constitue un résultat intermédiaire en pratique clinique, pour ainsi identifier une insuffisance rénale débutante et assurer le suivi d'une insuffisance rénale chronique ou après transplantation rénale en pratique clinique. Cette évaluation fine de la fonction rénale est aussi indispensable pour la réalisation de protocoles de recherche clinique et d'expérimentation animale.

Le recours à des méthodes de mesures précises du DFG, indice global de la fonction rénale, est indispensable du fait des nombreuses limitations liées à la seule estimation de ce paramètre physiologique par la clairance de la créatinine ou par la valeur de la créatininémie (marqueur endogène) (Froissart M. et al., Médecine Nucléaire, 1995, 19, 263-276 ; Médecine Nucléaire, 1995, 19, 251-262). Ces mesures font appel à des marqueurs de filtration glomérulaire. L'inuline reste le marqueur de référence mais les difficultés méthodologiques liées à son utilisation limitent son emploi à un petit nombre d'équipes expérimentées (Froissart et al., précités). Ainsi la méthode de la clairance rénale à l'équilibre de l'inuline avec recueil urinaire nécessite un personnel qualifié et disponible, la mise en place d'une perfusion continue et une vidange vésicale complète itérative pour chaque période, particulièrement difficile à mettre en oeuvre chez les sujets âgés et les jeunes enfants. Le calcul de la clairance à partir du débit de perfusion du marqueur ou traceur, réalisée à l'état d'équilibre, a tendance à surestimer de façon systématique la mesure de la clairance urinaire, du fait d'une sortie lente de l'inuline vers le secteur extra-cellulaire interstitiel, liée à une faible diffusibilité (Froissart et al. précités). De plus, l'inuline se prête mal aux méthodes de clairance après injection unique car l'atteinte de l'état d'équilibre entre les secteurs plasmatiques et interstitiels est tardive. Finalement, l'utilisation de l'inuline est limitée par des contraintes méthodologiques. Ainsi, la dissolution par chauffage dans un volume important est nécessaire avant administration d'inuline, et le dosage de l'inuline repose sur une méthode colorimétrique, pouvant poser problème en présence de chromogènes.

On peut aussi utiliser des éléments marqués (radiopharmaceutiques) éliminés par filtration glomérulaire comme le ⁵¹Cr-EDTA, le ^{99m}Tc-DTPA ou le ¹²⁵I-iothalamate. Le principal avantage des radiopharmaceutiques est la précision de leur mesure par comptage tant pour les prélèvements sanguins qu'urinaires. Leurs inconvénients multiples résident dans leur coût, la nécessité d'un laboratoire de radio-isotopes, et l'irradiation entraînée par leur injection (Froissart et al., précités).

Compte tenu de l'importance de la mesure du DFG en physiologie, en pathologie, en pharmacologie, et en recherche clinique et expérimentale, et des limitations de toutes les techniques à disposition du corps médical, le développement d'un nouveau marqueur de filtration glomérulaire, d'utilisation simple au lit du malade tout en permettant une mesure fiable du DFG est justifié.

Une substance peut être utilisée comme marqueur ou indicateur pour la mesure du DFG lorsqu'elle répond essentiellement aux critères suivants:
- être totalement filtrable par le glomérule,
- ne pas être sécrétée, ni réabsorbée ultérieurement dans le tubule,
- ne pas être métabolisée lors du passage dans le rein,
- ne pas avoir d'effet physiologique sur la fonction rénale,
- ne pas être liée aux protéines plasmatiques,
- ne pas être éliminée par voie extra-rénale,
- être dépourvue de toxicité et
- avoir une clairance constante pour une large plage de concentrations plasmatiques. (Smith H.W. et al., Smith HW., ed. *Principles of renal physiology.* New York: Oxford University Press, 1957, 25-35).

En conséquence, le Demandeur s'est donné pour but de pourvoir à un indicateur (marqueur ou traceur) de la filtration glomérulaire qui réponde mieux et plus facilement aux besoins de la pratique que les indicateurs de l'état de la technique (inuline, créatinine ou radiopharmaceutiques).

Le tétrapeptide hémorégulateur N-acétyl-Ser-Asp-Lys-Pro (acétyl-SDKP ou AcSDKP) est un inhibiteur naturel de l'entrée en phase S de la cellule souche hématopoïétique murine (Lenfant M. et al., Proc. Natl. Acad. Sci., 1989, **86**, 779-782). Chez la souris, l'administration exogène du peptide protège les cellules médullaires contre les effets cytotoxiques de médicaments anticancéreux et de l'irradiation et augmente significativement la survie des animaux traités (Masse A. et al., Blood, 1998, **91**, 441-9). Les propriétés biologiques de l'AcSDKP permettent notamment son utilisation comme nouvel agent hématoprotecteur susceptible d'être utilisé *in vivo* au cours de chimio- et radiothérapie anti-tumorale ainsi qu*'in vitro* comme un adjuvant des méthodes de purge de moelle osseuse dans le cas d'autogreffes chez l'homme (voir pour revue Azizi M. et al., Clin. Exp. Pharmacol. Physiol., 2001, **28**, 1066-1069). L'utilisation de l'AcSDKP chez l'homme a nécessité l'étude de sa stabilité dans les fluides biologiques. *In vitro,* l'enzyme de conversion de l'angiotensine I (ECA), qui possède 2 sites actifs N- et C-terminaux homologues et participe à la régulation de l'homéostasie cardiovasculaire, est la principale enzyme impliquée dans le catabolisme de l'AcSDKP (Rieger K.J. et al., Biochem. J., 1993, **296**, 1-6). Le site N-terminal de l'ECA est impliqué de façon préférentielle dans l'hydrolyse du peptide *in vitro* car son efficacité catalytique vis-à-vis de ce substrat est 50 fois supérieure à celle du site C-terminal (Rousseau A. et al., J. Biol. Chem., 1995, **270**, 3656-3661). Chez l'homme, les concentrations plasmatiques et urinaires d'AcSDKP augmentent, de manière significative, au cours de l'inhibition aiguë ou chronique de l'ECA (Azizi M. et al., J. Clin. Invest., 1996, **97**, 839-844). L'AcSDKP est épuré de l'organisme par deux mécanismes : l'hydrolyse par l'ECA d'une part, et la filtration glomérulaire, d'autre part (Azizi M. et al., Hypertension, 1999, **33**, 879-886). Lorsque l'ECA est active, la quantité d'AcSDKP mesurée dans l'urine est faible. Elle représente la quantité résiduelle d'AcSDKP intact, non dégradé par l'ECA plasmatique et endothéliale, qui est filtré au niveau des glomérules, puis dégradé par l'ECA présente à la surface des cellules tubulaires proximales. Quand l'hydrolyse du peptide est inhibée après administration d'un inhibiteur de l'ECA, on observe une augmentation massive de l'excrétion urinaire de l'AcSDKP qui est liée à deux mécanismes :
- à la filtration glomérulaire de la fraction libre d'AcSDKP intact dans le plasma,
- et surtout, à l'inhibition de l'ECA tubulaire par l'inhibiteur filtré au niveau du glomérule.

Sous inhibiteur de l'ECA, la clairance urinaire de l'AcSDKP est augmentée mais reste inférieure à la clairance de la créatinine endogène (69±27 %) (Azizi M. et al., 1999, précité). Cette différence est probablement due à l'intermittence de la dégradation du peptide par l'ECA entre les prises de l'inhibiteur et non à une réabsorption tubulaire du peptide, car l'AcSDKP est ionisé au pH urinaire et faiblement lié aux protéines plasmatiques (< 1%, Azizi M. et al., 1999, précité). En effet, la concentration d'AcSDKP plasmatique varie entre une valeur minimale de 2 pmol/ml et maximale de 4 à 6 pmol/ml entre deux prises de captopril 50 mg, et ce, de façon très parallèle à l'inhibition de l'ECA qui oscille entre 60 et 99 % entre 2 prises (Azizi M. et al., 1999, précité). Le parallélisme strict entre l'évolution des concentrations plasmatiques d'AcSDKP et l'activité de l'ECA démontre clairement une réactivation intermittente de l'ECA entre deux prises de captopril. La contribution exacte des deux mécanismes (clairance rénale et réactivation intermittente de l'ECA) apparaît clairement chez les patients ayant une insuffisance rénale chronique. En effet, on observe une accumulation majeure de l'AcSDKP dans le plasma chez les patients en insuffisance rénale, traités par inhibiteur de l'ECA (Azizi M. et al., 1999, précité). Les patients ayant une insuffisance rénale, non-traités par inhibiteur de l'ECA ont une discrète accumulation du peptide dans le plasma. Au cours de l'insuffisance rénale terminale, deux phénomènes concourent à l'augmentation massive des concentrations plasmatiques d'AcSDKP chez les patients traités par un inhibiteur de l'ECA :
- le peptide ne peut plus être éliminé par filtration glomérulaire,
- aucune réactivation intermittente de l'ECA ne peut avoir lieu, du fait de l'accumulation de l'inhibiteur de l'ECA dans le plasma et les tissus, en particulier chez les patients hémodialysés.

Dans le cadre de leurs travaux précédents, les Inventeurs ont en particulier observé que l'AcSDKP perfusé, en association avec un inhibiteur de l'ECA, pourrait servir de marqueur de la filtration glomérulaire (Azizi M. et al., J. Mol. Med., 2002, **80**, 492-8). Au cours d'une étude qui avait pour objectif d'évaluer les effets du polymorphisme insertion/délétion du gène de l'ECA sur le métabolisme de l'AcSDKP, les inventeurs ont perfusé l'AcSDKP à 1,12 µg/kg/min pendant 15 minutes à des volontaires sains qui recevaient pour la moitié d'entre eux un inhibiteur de l'ECA, le captopril à la dose de 50 mg 3 fois/jour pendant 3 jours et pour l'autre moitié, un placebo (Azizi M. et al., 2002, précité). Parmi les résultats obtenus, les Inventeurs montrent que sous inhibiteur de l'ECA, la clairance urinaire du peptide exogène est très proche du débit de filtration glomérulaire, estimée par la clairance de la créatinine endogène. Toutefois, cette procédure a l'inconvénient d'être complexe, puisque la perfusion du peptide doit être réalisée au pic de l'inhibition de l'ECA. De plus, il existe aussi une forte variabilité interindividuelle de l'inhibition de l'ECA liée à une biodisponibilité variable de l'inhibiteur.

Le Demandeur a ainsi sélectionné des marqueurs ou indicateurs qui répondent aux conditions énoncées ci-dessus tout en ne présentant pas les inconvénients des méthodes antérieures décrites. Elle a trouvé que, de manière inattendue, les analogues du tétrapeptide hémorégulateur N-acétyl-Ser-Asp-Lys-Pro (AcSDKP) résistants à l'ECA sont adaptés à une utilisation comme marqueurs, traceurs ou indicateurs spécifiques de filtration glomérulaire et répondent ainsi mieux aux besoins de la pratique que les méthodes antérieurement utilisées pour mesurer le débit de filtration glomérulaire.

En conséquence, la présente invention a pour objet l'utilisation des analogues du N-acétyl-Ser-Asp-Lys-Pro, résistants à l'ECA, pour la préparation d'un réactif ou traceur adapté à la mesure du DFG.

Les analogues du N-acétyl-Ser-Asp-Lys-Pro, résistants à l'ECA et leurs procédés de préparation sont notamment décrits dans les articles au nom de Gaudron S. et al. (*Stem Cells,* 1999, 17, 2,100-106 ; *J. Med. Chem.,* 1997, 40, 24, 3963-3968) ; ces analogues sont les suivants :
- trois pseudopeptides, dans lesquels l'une des liaisons peptidiques a été remplacée par un groupe amino-méthylène Ψ(CH₂-NH) :
   N-acétyl-Ser (CH₂-NH)-Asp-Lys-Pro: SΨ
   N-acétyl-Ser-Asp (CH₂-NH)-Lys-Pro : DΨ
   N-acétyl-Ser-Asp-Lys(CH₂-NH)-Pro: KΨ et
- un peptide modifié à son extrémité C-terminale par amidation : N-acétyl-Ser-Asp-Lys-Pro-NH₂. Ce produit ne diffère de la molécule initiale que par amidification de la fonction carboxyle du résidu proline situé en position C-terminale du peptide.

Ces différentes modifications confèrent auxdits analogues de l'AcSDKP une grande stabilité vis-à-vis de l'hydrolyse induite par l'ECA.

Conformément à ladite utilisation, lesdits analogues du N-acétyl-Ser-Asp-Lys-Pro, résistants à l'ECA sont avantageusement radiomarqués.

La présente invention a également pour objet un procédé de mesure du DFG, caractérisé en ce qu'il comprend la détermination de la clairance plasmatique et/ou la mesure de la clairance rénale à l'état d'équilibre de l'un desdits analogues du N-acétyl-Ser-Asp-Lys-Pro après injection intravasculaire, de préférence unique, dudit analogue.

De manière surprenante, la mise en oeuvre de ces analogues du N-acétyl-Ser-Asp-Lys-Pro, pourrait permettre en déterminant la clairance plasmatique totale (Ct) ou, de préférence, en calculant par une méthode simplifiée, la valeur de la clairance plasmatique (C) selon des méthodes connues en soi et notamment décrites dans les articles aux noms de Froissart et al. (précités), d'éviter les mesures de clairance urinaire fractionnées, plus lourdes à mettre en oeuvre chez l'homme [mais qui peuvent néanmoins être utiles, chez l'animal, et dans le cadre d'études cliniques] et de réduire le nombre de prélèvements à quelques points et même à un seul point, tout en ne présentant pas les inconvénients des radiopharmaceutiques.

Selon un mode de mise oeuvre avantageux dudit procédé, l'analogue du N-acétyl-Ser-Asp-Lys-Pro est sélectionné dans le groupe constitué par le N-acétyl-Ser (CH₂-NH)-Asp-Lys-Pro (SΨ), le N-acétyl-Ser-Asp (CH₂-NH)-Lys-Pro (DΨ), le N-acétyl-Ser-Asp-Lys(CH₂-NH)-Pro (KΨ) et le N-acétyl-Ser-Asp-Lys-Pro-NH₂.

Conformément à l'invention, la mesure de la concentration dudit analogue du N-acétyl-Ser-Asp-Lys-Pro est effectuée par tout moyen de détection sensible et spécifique dudit analogue dans au moins un échantillon biologique et notamment par immunodosage ou par les techniques de chromatographie et/ou de spectrométrie de masse.

De manière avantageuse, ledit immunodosage est un immunodosage très spécifique, n'ayant pas de réaction croisée ni avec la molécule naturelle (AcSDKP) ni avec d'autres peptides amidés ni avec des inhibiteurs de l'enzyme de conversion (Junot C. et al., J. Immunoassay Immunochem., 2001, **22**, 15-31). Cette technique de dosage par EIA (immunoessai enzymatique) a une bonne précision et une bonne reproductibilité. Sa limite de quantification est de 1 nM dans le plasma. Par ailleurs, AcSDKP-NH₂ n'est pas dégradé par l'ECA présente dans le plasma de souris (Junot C. et al., 2001 précité). Sa stabilité évaluée dans les conditions expérimentales et de réalisation des dosages est satisfaisante. De plus, aucune altération n'a été constatée après 3 cycles de congélation-décongélation. Les coefficients de variation (CV) de répétabilité, de reproductibilité sont compris entre 1,4 et 18% et les valeurs d'exactitude sont comprises entre 85 et 115% des valeurs théoriques (Junot C. et al., 2001 précité).

Egalement conformément à l'invention, ledit analogue du N-acétyl-Ser-Asp-Lys-Pro peut être radiomarqué. Dans ce cas, la mesure de la concentration en peptide est réalisée par une technique d'imagerie ou par comptage radioactif des échantillons.

La présente invention a également pour objet un kit ou coffret de mesure du débit de filtration glomérulaire, caractérisé en ce qu'il comprend un analogue du N-acétyl-Ser-Asp-Lys-Pro sélectionné dans le groupe constitué par le N-acétyl-Ser (CH₂-NH)-Asp-Lys-Pro (SΨ), le N-acétyl-Ser-Asp (CH₂-NH)-Lys-Pro (DΨ), le N-acétyl-Ser-Asp-Lys(CH₂-NH)-Pro (KΨ) et le N-acétyl-Ser-Asp-Lys-Pro-NH₂ et un moyen de détection dudit analogue dans au moins un échantillon biologique.

Selon un mode de réalisation avantageux dudit kit ou coffret, ledit moyen de détection dudit analogue est sélectionné dans le groupe constitué par les immunodosages, les techniques de chromatographie et/ou de spectrométrie de masse.

Selon un autre mode de réalisation avantageux dudit kit ou coffret, ledit analogue est radiomarqué et ledit moyen de détection est sélectionné dans le groupe constitué par les techniques d'imagerie et les techniques de comptage radioactif.

Les analogues du N-acétyl-Ser-Asp-Lys-Pro répondent aux critères de choix d'un marqueur ou indicateur idéal de la filtration glomérulaire :
- ils sont librement filtrés,
- ils ne se lient pas aux protéines plasmatiques,
- il ne sont ni sécrétés, ni réabsorbés ni métabolisés par le tubule,
- ils sont sans effet physiologique lors d'une administration unique,
- ils sont dépourvus de toxicité et sans élimination par voir extra rénale,
- ils résistent à l'hydrolyse par l'ECA de rat et humaine, *in vitro* et *in vivo* contrairement au peptide naturel.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

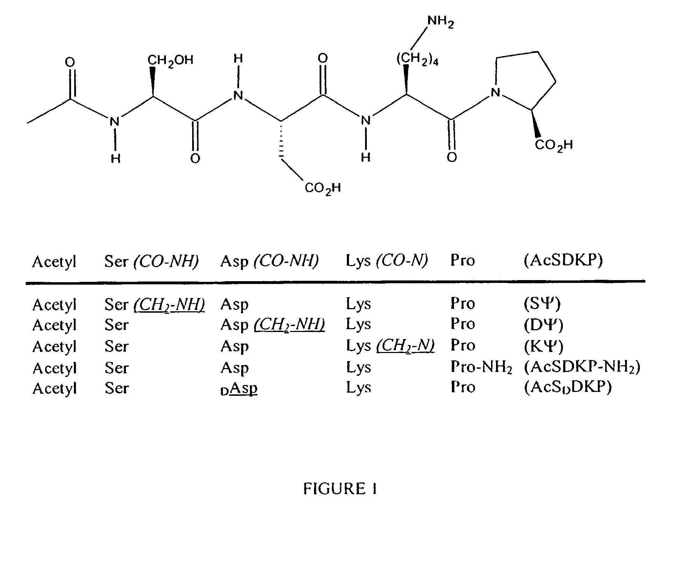

- la figure 1 représente les formules des analogues du N-Acétyl-Ser-Asp-Lys-Pro

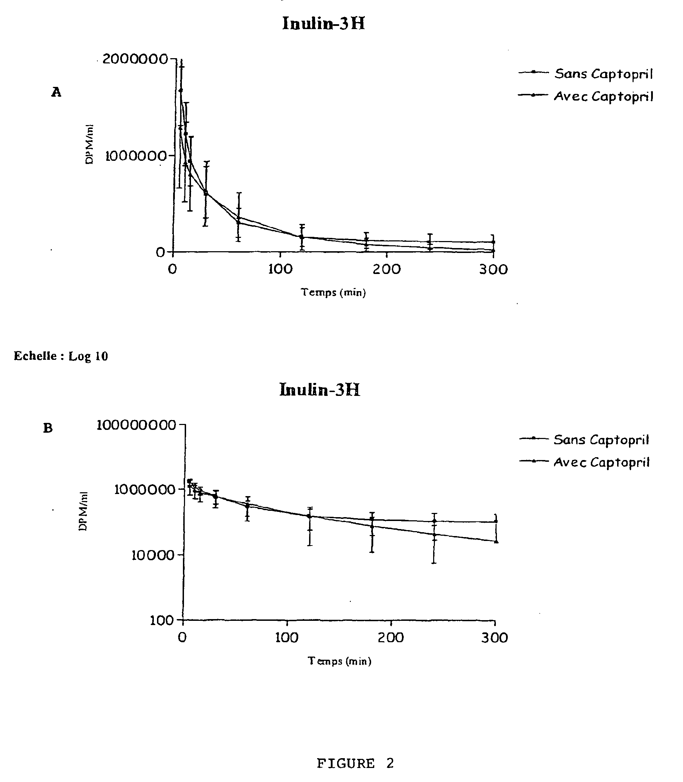

- les figures 2A et 2B représentent la cinétique plasmatique de l'inuline avec et sans captopril

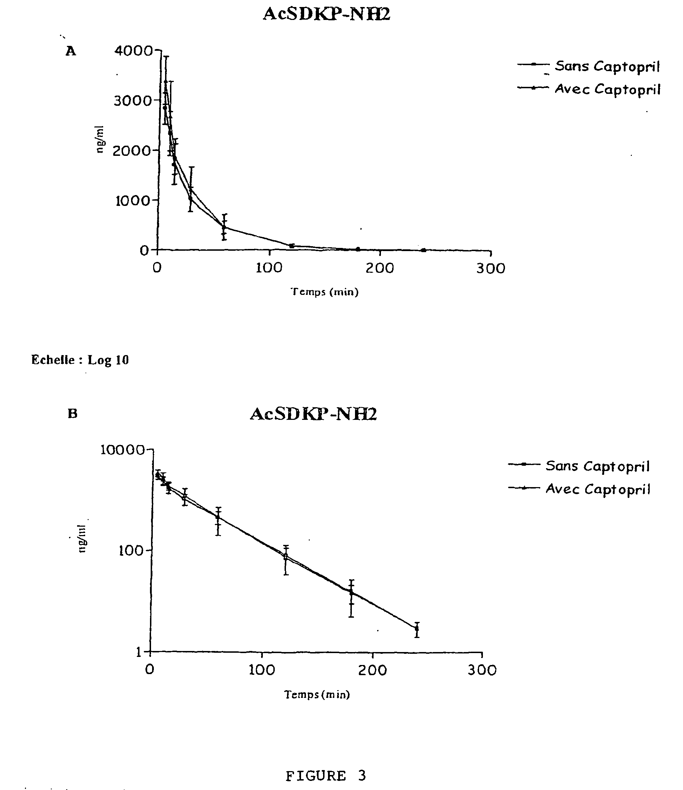

- les figures 3A et 3B représentent la cinétique plasmatique de l'analogue N-Acétyl-Ser-Asp-Lys-Pro-NH₂, avec et sans captopril.

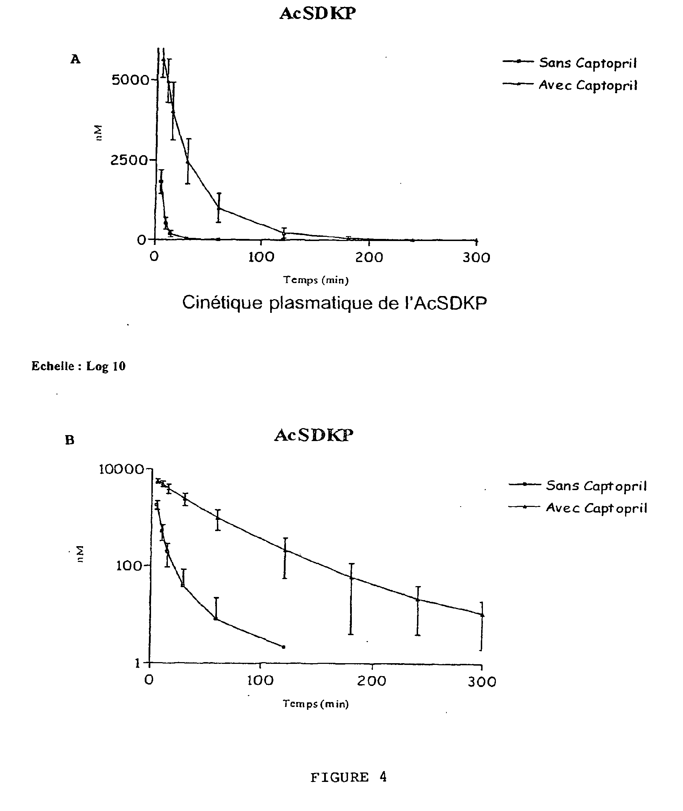

- les figures 4A et 4B représentent la cinétique plasmatique du N-Acétyl-Ser-Asp-Lys-Pro, avec et sans captopril.

Il doit être bien entendu, toutefois, que ces exemples sont donnés uniquement à titre d'illustration de l'objet de l'invention, dont ils ne constituent en aucune manière une limitation.

### EXEMPLE 1:

### I Matériels et Méthodes

### I.1. Produits

Les deux peptides (AcSDKP et AcSDKP-NH₂ - 97 % de pureté) proviennent de la société NEOSYSTEME. Le captopril et l'Inuline-³H (95 % de pureté-Activité spécifique : 200 mCi/g) proviennent de la société SIGMA. Chaque solution d'injection a été préparée séparément en eau physiologique un jour avant les expérimentations animales.

Le contrôle de la concentration des solutions administrées ainsi que le dosage des échantillons ont été réalisés par dosage immunologique, conformément à la méthode exposée dans Junot C. et al., précité pour les groupes traités avec l'AcSDKP et avec l'AcSDKP-NH₂ et par scintillation liquide pour les groupes témoins traités avec de l'Inuline-³H (G).

La limite de détection choisie pour le groupe AcSDKP est de 0,2 nM pour les échantillons plasmatiques et de 0,5 nM pour les échantillons urinaires. Pour le groupe AcSDKP-NH₂ la limite de détection est de 0,15 ng/ml pour les échantillons plasmatiques et en urinaires.

### I.2. Animaux

L'étude a été réalisée sur des rats mâles Wistar (poids moyen environ 320 g) préalablement cathéthérisés au niveau de l'artère fémorale. Le nombre de rats était de cinq par groupe (6 groupes).
- Groupe 1 : 1 mg/kg d'AcSDKP en I.V. (bolus),
- Groupe 2 : captopril (10 mg/kg/j) pendant 7 jours par gavage puis 1 mg/kg d'AcSDKP + 1 mg/kg de captopril en I.V. (bolus) le jour de la cinétique,
- Groupe 3 : 1 mg/kg d'AcSDKP-NH₂ en I.V. (bolus),
- Groupe 4 : captopril (10 mg/kg/j) pendant 7 jours par gavage puis 1 mg/kg d'AcSDKP-NH₂ + 1 mg/kg de captopril en I.V. (bolus) le jour de la cinétique,
- Groupe 5 : 1 mg/kg d'inuline-³H(G) (environ 78 µci/rat) en I.V. (bolus),
- Groupe 6 : captopril (10 mg/kg/j) pendant 7 jours par gavage puis 1 mg/kg d'inuline-³H(G) (environ 60 µci/rat) en I.V. (bolus).

### Déviations au protocole :

- Un rat supplémentaire a été pris en compte pour le groupe n°3 (AcSDKP-NH₂ seul)
- Doses administrées : 1,20 mg/kg pour l'AcSDKP - 0,82 mg/kg pour l'AcSDKP-NH₂ - 1,05 mg/kg pour l'inuline -³H
- Prélèvements urinaires : des prélèvements supplémentaires ont été réalisés pour les groupes (1-2-3-4-5) afin d'avoir un pool d'informations le plus important possible :
   Groupe n°1 : T₀₋₂ₕ - T₂₋₄ₕ - T₄₋₅ₕ - T₅₋₂₄ₕ - T₂₄₋₃₆ₕ - T₃₆₋₄₈ₕ
   Groupe n°2 : T₂₄₋₃₀ₕ - T₃₀₋₄₈ₕ
   Groupe n°3 : T₄₋₇ₕ - T₇₋₂₄ₕ - T₂₄₋₄₈ₕ
   Groupe n°4 : T₀₋₄ₕ - T₄₋₂₄ₕ - T₂₄₋₄₈ₕ - T₄₈₋₇₂ₕ
   Groupe n°5 : T₂₋₅ₕ - T₅₋₇ₕ - T₇₋₂₄ₕ - T₂₄₋₃₀ₕ - T₃₀₋₄₈ₕ - T₄₈₋₇₂ₕ

### I.3. Mesures

La concentration plasmatique des composés a été mesurée aux temps suivants : T₀- T₅- T₁₀- T₃₀ T₆₀- T₁₂₀ - T₂₄₀ - T₃₀₀ minutes.

La concentration urinaire des composés a été mesurée aux temps suivants : T₋₂₄₋₀ₕ (avant le début des expériences), T₀₋₂ₕ, T₂₋₄ₕ, T₄₋₂₄ₕ et aux temps décrits dans la partie « déviations au protocole » pour les prélèvements urinaires supplémentaires.

### II Analyse pharmacocinétique

Les principaux paramètres pharmacocinétiques obtenus par modélisation sur logiciel Siphar V04 (Simed, Créteil) ont été :
- AUC_{0-Tlast}: aire sous la courbe jusqu'au dernier point mesurable
- AUC_{0-inf} : aire sous la courbe jusqu'à l'infini
- T ½ : demi-vie d'élimination
- clairance plasmatique
- clairance urinaire : (quantité urinaire excrétée totale/AUC plasmatique)/poids du rat

La modélisation pharmacocinétique a été réalisée de la manière suivante:
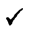 Analyse bicompartimentale pour les groupes traités avec l'inuline-³H
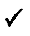 Analyse monocompartimentale pour les groupes traités avec l'AcSDKP et l'AcSDKP-NH₂.

### III Résultats

Les concentrations plasmatiques et urinaires mesurée ainsi que les paramètres pharmacocinétiques modélisées pour chaque rat sont présentés :

| | | |
|---|---|---|
| Tableau I | AcSDKP | groupe n°1 |
| Tableau II | AcSDKP en présence de captopril | groupe n°2 |
| Tableau III | AcSDKP-NH₂ | groupe n°3 |
| Tableau IV | AcSDKP-NH₂ en présence de captopril | groupe n°4 |
| Tableau V | Inuline-³H | groupe n°5 |
| Tableau VI | Inuline-³H en présence de captopril | groupe n°6 |
| Tableau VII | Tableau Récapitulatif | |

L'AcSDKP est un peptide endogène chez le rat. De par ce fait, pour les groupes n°1 et n°2, l'AcSDKP endogène a dû être retranché des résultats. Le taux d'AcSDKP endogène a été déterminé en faisant la somme de la quantité d'AcSDKP (nmol) de la période T₂₄₋₄₈ₕ (on estime que 24 heures après l'administration du peptide en I.V., seul l'AcSDKP endogène est présent). Ce résultat a été multiplié par 2 (pour la période T₀₋₂₄ₕ et T₂₄₋₄₈ₕ) et retranché au total de la quantité urinaire pour chaque rat.

Les représentations graphiques des concentrations plasmatiques moyennes de chaque groupe sont représentées :

| | | |
|---|---|---|
| Figures 2A et 2B | Inuline-³H | Groupes n°5 et n°6 |
| Figures 3A et 3B | AcSDKP-NH₂ | Groupes n°3 et n°4 |
| Figures 4A et 4B | AcSDKP | Groupes n°1 et n°2 |

**Tableau I - Groupe n°1 - AcSDKP sans captopril Concentration plasmatique, urinaire et paramètres pharmacocinétiques pour l'AcSDKP**

| **Concentration plasmatique (nM)** | | | | | | | |
|---|---|---|---|---|---|---|---|
| | **rats** | | | | | | |
| temps (min) | 1 | 3 | 4 | 5 | 8 | **moyenne** | **SD** |
| 5 | 2188 | 1759 | 1239 | 2100 | 1802 | **1818** | **373** |
| 10 | 729 | 400 | 265 | 671 | 508 | **514** | **191** |
| 15 | 268 | 96 | 135 | 328 | 143 | **194** | **99** |
| 30 | 34 | 14 | 10 | 121 | 10 | **38** | **47,6** |
| 60 | 5 | 0,36 | 0,15 | 33 | 2 | **8,2** | **14,2** |
| 120 | ILQ | ILQ | ILQ | 2 | ILQ | **2,2** | **-** |
| 180 | ILQ | ILQ | ILQ | ILQ | ILQ | **-** | **-** |
| 240 | ILQ | ILQ | ILQ | ILQ | ILQ | **-** | **-** |
| 300 | ILQ | ILQ | ILQ | ILQ | ILQ | **-** | **-** |
| AUC_{0-inf} nM*min | 230401 | 16319 | 13026 | 18178 | 16223 | **17393** | **3625** |
| t1/2 (min) | 6,4 | 4,7 | 4,4 | 12,8 | 5,9 | **6,8** | **3,4** |
| VD (ml/kg) | 894 | 1070 | 1034 | 2600 | 1346 | **1389** | **697** |

| Concentration urinaire (nM) | | | | | | | |
|---|---|---|---|---|---|---|---|
| 0-2h | 22830 | NS | 27250 | 135 | NS | | |
| 2-4h | 11810 | NS | 16220 | 14270 | NS | | |
| 4-5h | 12050 | 14060 | NS | 7806 | NS | | |
| 5-24h | 299 | 2494 | NS | 965 | 1334 | | |
| 21-36h | 244 | 333 | 2697 | 93 | NS | | |
| 36-48h | 77 | 61 | 214 | 55 | 141 | | |

| Volume urinaire (ml) | | | | | | | |
|---|---|---|---|---|---|---|---|
| 0-2h | 1.1 | NS | 1,7 | 0,7 | NS | | |
| 2-4h | 0,6 | NS | 1,0 | 2,5 | NS | | |
| 4-5h | 2,8 | 3,7 | NS | 1,9 | NS | | |
| 5-24h | 33,2 | 4,1 | NS | 21,3 | 58,9 | | |
| 24-36h | 16,9 | 10.1 | 5,2 | 9,' | NS | | |
| 36-48h | 51,6 | 27.1 | 16,1 | 36,7 | 56,1 | | |

| Q urinaire (nmol) | | | | | | | |
|---|---|---|---|---|---|---|---|
| 0-2h | 25,1 | 0,0 | 46,7 | 0,1 | NS | | |
| 2-4h | 7,1 | 0,0 | 15,7 | 36,4 | NS | | |
| 4-5h | 33,5 | 51,3 | 0,0 | 14,6 | NS | | |
| 5-24h | 9,9 | 10,2 | 0,0 | 20,6 | 78,5 | | |
| 24-36h | 4,1 | 3,4 | 15,5 | 0,9 | NS | | |
| 36-48h | 4,0 | 1,7 | 3,4 | 2,0 | 7,9 | | |
| total (nmol) | 83,7 | 66,6 | 81,3 | 74,5 | 86,5 | | |
| Q administrée (nmol) | 757,7 | 706,5 | 771,9 | 833 | 832,0 | | |
| poids (kg) | 0,3392 | 0,3265 | 0,3035 | 0,3210 | 0,3258 | **0,32** | **0,01** |
| dose (mg/kg) | 1,09 | 1,05 | 1,24 | 1,27 | 1,24 | **1,18** | **0,1** |
| % excrété | 11 | 9,4 | 10,5 | 8,9 | 10,4 | **10,07** | **0,86** |
| % excrété - endog | 8,9 | 8,0 | 5,6 | 8,3 | 8,5 | **7,86** | **1,29** |
| CL_{R} (ml/min/kg) | 10,7 | 12,5 | 20,3 | 12,8 | 16,4 | **14,52** | **3,82** |
| CL_{R}(ml/min/kg)-endog | 8,6 | 11,1 | 11.5 | 11,8 | 13,0 | **11,21** | **1,61** |
| Ctotale (ml/min/kg)dose/AUC | 97 | 132,6 | 192,6 | 142,8 | 157,4 | **144,46** | **34,95** |

| | | | | | | | |
|---|---|---|---|---|---|---|---|
| NS : pas d'échantillon | | | | | | | |

**Tableau II - Groupe n°2 - AcSDKP avec captopril Concentration plasmatique, urinaire et paramètres pharmacocinétiques pour l'AcSDKP en présence de Captopril**

| **Concentration plasmatique (nM)** | | | | | | | |
|---|---|---|---|---|---|---|---|
| | **rats** | | | | | | |
| temps (min | 1 | 2 | 6 | 7 | 8 | **moyenne** | **SD** |
| 5 | 6707 | 5413 | 5511 | 5293 | 5393 | **5663** | **588** |
| 10 | 5920 | 4813 | 4288 | 5439 | 4445 | **4981** | **687** |
| 15 | 5571 | 4016 | 3399 | 3876 | 3343 | **4041** | **904** |
| 30 | 3645 | 2176 | 2485 | 2173 | 1804 | **2457** | **707** |
| 60 | 1759 | 735 | 848 | 1069 | 590 | **1000** | **459** |
| 120 | 501 | 151 | 145 | 197 | 97 | **218** | **162** |
| 180 | 159 | 32 | 39 | 48 | 21 | **60** | **56** |
| 240 | 52 | 10 | 18 | 23 | 7 | **22** | **18** |
| 300 | 26 | 9 | 6 | 11 | 4 | **11** | **9** |
| AUC 0-INF | 341133 | 196456 | 200683 | 217932 | 167504 | **224742** | **67543** |
| nM*min | | | | | | | |
| t1/2 (min) | 35,5 | 29,2 | 29,5 | 31,4 | 28,1 | **30,7** | **2,9** |
| Vd(ml/kg) | 353 | 653 | 499 | 505 | 567 | 515 | 110 |

| Concentration urinaire (nM) | | | | | | | |
|---|---|---|---|---|---|---|---|
| 0-2h | NS | NS | NS | NS | NS | | |
| 2-4h | 253 | 821500 | 126214 | NS | 126118 | | |
| 4-24h | 25502 | 2119 | 7607 | 58597 | 5746 | | |
| 24-30h | 202 | 1081 | NS | NS | NS | | |
| 30-48h | 248 | 55 | 197 | 69 | 165 | | |

| Volume urinaire (ml) | | | | | | | |
|---|---|---|---|---|---|---|---|
| 0-2h | NS | NS | NS | NS | NS | | |
| 2-4h | 0,8 | 0,9 | 6,5 | NS | 4.2 | | |
| 4-24h | 19,8 | 23,1 | 28,9 | 11,1 | 35,2 | | |
| 24-30h | 8,2 | 8,9 | NS | NS | NS | | |
| 30-48h | 32,4 | 44,3 | 53,9 | 6,5 | 64,4 | | |
| total | 61,1 | 77,2 | 89,2 | 17,6 | 103,9 | | |

| Q urinaire (nmol) | | | | | | | |
|---|---|---|---|---|---|---|---|
| 0-2h | 0,0 | 0,0 | 0,0 | 0,0 | 0,0 | | |
| 2-4h | 0,2 | 744,4 | 820,9 | 0,0 | 535,3 | | |
| 4-6h | 0,0 | 0,0 | 0,0 | 0.0 | 0,0 | | |
| 6-24h | 504,6 | 49,0 | 219,7 | 649,1 | 202,3 | | |
| 24-30h | 1,6 | 9,6 | 0,0 | 0,0 | 0,0 | | |
| 30-48h | 8,0 | 2,5 | 10,6 | 0,4 | 10,6 | | |
| total (nmol) | 514,5 | 805,5 | 1051,2 | 649,5 | 748,3 | | |
| Q administrée (nmol) | 657,7 | 918.1 | 739,4 | 718.9 | 764,7 | | |
| poids (kg) | 0,2796 | 0,3017 | 0,3135 | 0,2955 | 0,3211 | **0,30** | **0,02** |
| dose (mg/kg) | 1,15 | 1,48 | 1,15 | 1.19 | 1,16 | **1,23** | **0,15** |
| % excrété | 78,2 | 87,7 | 142,2 | 90,4 | 97,9 | **99,27** | **25,00** |
| % excrété - endog | 75,3 | 85,1 | 139,3 | 90,2 | 95.1 | **97,00** | **24,77** |
| CL_{R} (ml/min/kg) | 5,39 | 13,59 | 16,71 | 10,09 | 13.91 | **11,94** | **4,35** |
| CL_{R} (ml/min/kg)-endog | 5,2 | 13,2 | 16,4 | 10,1 | 13,5 | **11,67** | **4,25** |
| Ctotale (ml/min/kg)dose/AUC | 6,9 | 15,5 | 11,8 | 11,2 | 14,2 | **11,9** | **3,31** |

| | | | | | | | |
|---|---|---|---|---|---|---|---|
| NS : pas d'échantillon | | | | | | | |

**Tableau III - Groupe n°3 - AcSDKP- NH₂ avec Captopril Concentration plasmatique, urinaire et paramètres pharmacocinétiques pour l'AcSDKP-NH₂**

| **Concentration plasmatique (ng/ml)** | | | | | | | | |
|---|---|---|---|---|---|---|---|---|
| | **rats** | | | | | | | |
| temps (min) | 1 | 2 | 1 bis | 4 | 5 | 6 | **moyenne** | **SD** |
| 5 | 2581 | 3139 | 2865 | 2491 | 3250 | 2663 | **2831** | **309** |
| 10 | 1611 | 2288 | 2425 | 2152 | 2918 | 2595 | **2331** | **442** |
| 15 | 1655 | 985 | 2204 | 1796 | 1930 | 1750 | **1720** | **407** |
| 30 | 748 | 880 | 1317 | 1329 | 930 | 867 | **1012** | **248** |
| 60 | 233 | 443 | 598 | 545 | 416 | 463 | **450** | **126** |
| 120 | 47 | 45 | 163 | 118 | 48 | 69 | **82** | **48** |
| 180 | 6 | 8 | 25 | 32 | 9 | 19 | **16** | **11** |
| 240 | ILQ | ILQ | 3 | 5 | 2 | 4 | **3** | **1** |
| 300 | ILQ | ILQ | ILQ | ILQ | ILQ | ILQ | | |
| AUC_{0-INF} ng/ml*min | 73978 | 87407 | 125025 | 111894 | 101003 | 96190 | **99249** | **17976** |
| t1/2 (min) | 20,7 | 20,7 | 24,5 | 26,8 | 21,8 | 25,1 | **23,3** | **2,5** |
| Vd(ml/kg) | 338 | 270 | 220 | 253 | 256 | 261 | **266** | **39** |

| Concentration urinaire (ng/ml) | | | | | | | | |
|---|---|---|---|---|---|---|---|---|
| 0-2h | 41655 | NS | NS | NS | NS | NS | | |
| 2-4h | NS | 67056 | NS | 22941 | 148362 | NS | | |
| 4-7h | 19076 | 9228 | NS | NS | NS | NS | | |
| 7-24h | 462 | 192 | 8467 | 965 | 815 | 6202 | | |
| 24-48h | 10 | 42 | 986 | 16 | ILQ | 59 | | |

| Volume urinaire (ml) | | | | | | | | |
|---|---|---|---|---|---|---|---|---|
| 0-2h | 4,22 | NS | NS | NS | NS | NS | | |
| 2-4h | NS | 2,69 | NS | 6,16 | 1,96 | NS | | |
| 4-7h | 4,41 | 2,55 | NS | NS | NS | NS | | |
| 7-24h | 34,69 | 44,55 | 24,98 | 43,42 | 35,87 | 25,7 | | |
| 24-48h | 53,33 | 31,19 | 47,91 | 37,26 | 64,86 | 53,41 | | |

| Q urinaire (ng) | | | | | | | | |
|---|---|---|---|---|---|---|---|---|
| 0-2h | 175685,1 | 0,0 | 0,0 | 0,0 | 0,0 | 0,0 | | |
| 2-4h | 0,0 | 180325.8 | 0,0 | 141210,2 | 290581,1 | 0.0 | | |
| 4-7h | 84030,9 | 23548,7 | 0,0 | 0,0 | 0,0 | 0,0 | | |
| 7-24h | 16014,8 | 8570,5 | 211497,2 | 41898,9 | 29223,7 | 159391,7 | | |
| 24-48h | 513,0 | 1304,8 | 47218,0 | 603,6 | ILQ | 3161,4 | | |
| total (ng) | 276243,9 | 213749,7 | 258715,2 | 183712,7 | 319804,8 | 162553,1 | | |
| Q administrée (ng) | 264881 | 251981 | 253401 | 246391 | 264881 | 233921,0 | | |
| poids (kg) | 0,3162 | 0,3196 | 0.3253 | 0,3363 | 0,3221 | 0,3375 | **0,33** | **0,01** |
| dose (mg/kg) | 0,84 | 0,79 | 0,78 | 0,73 | 0,82 | 0,69 | **0,78** | **0,05** |
| % excrété | 104,3 | 84,8 | 102,1 | 74,6 | 120,7 | 69,5 | **92,67** | **19,69** |
| CL_{R} (milminikg) | 11,8 | 7,7 | 6,4 | 4,9 | 9,8 | 5,0 | **7,59** | **2,77** |
| Ctotale (ml/min/kg)dose/AUC | 11,3 | | 6.2 | 6,5 | 8,1 | 7,2 | **8,08** | **1,89** |

| | | | | | | | | |
|---|---|---|---|---|---|---|---|---|
| NS : pas d'échantillon | | | | | | | | |

**Tableau IV - Groupe n°4 - AcSDKP-NH₂ avec Captopril Concentration plasmatique, urinaire et paramètres pharmacocinétiques pour l'AcSDKP-NH₂ en présence de captopril**

| **Concentration plasmatique (ng/ml)** | | | | | | | |
|---|---|---|---|---|---|---|---|
| | **rats** | | | | | | |
| temps (min) | 1 | 2 | 3 | 4 | 6 bis | **moyenne** | **SD** |
| 5 | 3623 | 3115 | 4099 | 2863 | 3280 | **3396** | **480** |
| 10 | 1750 | 3571 | 3075 | 2682 | 2346 | **2685** | **694** |
| 15 | 1432 | 1978 | 2363 | 1635 | 1960 | **1874** | **357** |
| 30 | 1128 | 891 | 1173 | 889 | 1986 | **1213** | **452** |
| 60 | 312 | 338 | 346 | 357 | 910 | **453** | **256** |
| 120 | 31 | 63 | 64 | 73 | 140 | **74** | **40** |
| 180 | 7 | 11 | 18 | 16 | 23 | **15** | **6** |
| 240 | 2 | 3 | 3 | 3 | 2 | **3** | **0** |
| 300 | ILQ | ILQ | ILQ | ILQ | 0 | **0** | |
| AUC _{0-INF} ng/ml*min | 89343 | 100543 | 114983 | 92278 | 153339 | **110097** | **26140** |
| t1/2 (min) | 21,9 | 23,0 | 23,1 | 24,1 | 23,3 | **23,1** | **0,8** |
| Vd(ml/kg) | 284 | 230 | 260 | 327 | 242 | **269** | **38** |

| Concentration urinaire (ng/ml) | | | | | | | |
|---|---|---|---|---|---|---|---|
| 0-4h | NS | NS | NS | NS | NS | | |
| 4-24h | 10906 | 3107 | 11032 | 22749 | 10380 | | |
| 24-48h | 239 | 35 | 337 | 171 | 178 | | |
| 48-72h | 11 | ILQ | 11 | 70 | NS | | |

| Volume urinaire (ml) | | | | | | | |
|---|---|---|---|---|---|---|---|
| 0-4h | 0,00 | 0,00 | 0,00 | 0,00 | 0,00 | | |
| 4-24h | 19,12 | 46,46 | 25,93 | 12,05 | 34,09 | | |
| 24-48h | 44,93 | 62,22 | 48,50 | 58,89 | 37,78 | | |
| 48-72h | 70,88 | 57,86 | 58,98 | 57,25 | 0,00 | | |

| Q urinaire (ng) | | | | | | | |
|---|---|---|---|---|---|---|---|
| 0-4h | | | | | | | |
| 4-24h | 208504,2 | 144361,5 | 286064,8 | 274157,3 | 353834,5 | | |
| 24-48h | 10737,7 | 2183,9 | 16360,4 | 10087,6 | 6728,5 | | |
| 48-72h | 758,4 | ILQ | 619,3 | 4008,3 | 0,0 | | |
| total (ng) | 220000 | 146545,0 | 303045 | 288253 | 360563 | | |
| Q administrée (ng) | 242311 | 216121 | 259558 | 255512 | 341274 | **262955,22** | |
| poids (kg) | 0,3009 | 0,3100 | 0,2900 | 0,2951 | 0,3092 | **0,30** | **0,01** |
| dose (mg/kg) | 0,81 | 0,7 | 0,9 | 0,87 | 1,1 | **0,87** | **0,15** |
| % excrété | 90,8 | 67,8 | 116,8 | 112.8 | 105,7 | **98,76** | **19,94** |
| CL_{R} (ml/min/kg) | 8,2 | 4,7 | 9,1 | 10,6 | 7,6 | **8,03** | **2,18** |
| Ctotale (ml/min/kg)dose/AUC | 9.0 9,0 | 6,9 6,9 | 7,8 7,8 | 9,4 9,4 | 7,2 7,2 | **8,06 8,06** | **1,09 1,09** |

| | | | | | | | |
|---|---|---|---|---|---|---|---|
| NS : Pas d'échantillon | | | | | | | |

**Tableau V - Groupe n°5 - Inuline-³NH sans Captopril Concentration plasmatique, urinaire et paramètres pharmacocinétiques pour l'Inuline-³H**

| **Concentration plasmatique (DPM/ml)** | | | | | | | |
|---|---|---|---|---|---|---|---|
| | **rats** | | | | | | |
| temps (min) | 6 | 7 | 8 | 9 | 10 | **moyenne** | **SD** |
| 5 | 1,44E+06 | 2.10E+08 | 1,93E+06 | 1,67E+06 | 1,21E+06 | **1,67E+06** | **3,60E+05** |
| 10 | 1,31E+06 | 1,62E+06 | 1,38E+06 | 1,01E+06 | 7,85E+05 | **1,22E+06** | **3,25E+08** |
| 15 | 1,15E+06 | 1,14E+06 | 1,07E+06 | 7,84E+05 | 5,65E+05 | **9,41E+05** | **2,57E+05** |
| 30 | 9,86E+05 | 7,67E+05 | 6,05E+05 | 3,95E+05 | 3,38E+05 | **6,18E+05** | **2,67E+05** |
| 60 | 5,00E+05 | 4,03E+05 | 2,97E+05 | 1,58E+05 | 1,64E+05 | **3,04E+05** | **1,49E+05** |
| 120 | 2,52E+05 | 2,19E+05 | 1,98E+05 | 5,80E+04 | 4,33E+04 | **1,54E+05** | **9,65E+04** |
| 180 | 1,94E+05 | 1,77E+05 | 1,71E+05 | 4,17E+04 | 2,45E+04 | **1,21E+05** | **8,13E+04** |
| 240 | 1.72E+05 | 1,59E+05 | 1,74E+05 | 2,76E+04 | 1,56E+04 | **1,09E+05** | **8,06E+04** |
| 300 | 1,55E+05 | 1,78E+05 | 1,57E+05 | 2,25E+04 | 1,38E+04 | **1,05E+05** | **8,01 E+04** |
| AUC_{0-Inf}-DPM/ml*min | 1,75E+08 | 1,47E+08 | 1,64E+08 | 5,40E+07 | 4,22E+07 | **1,16E+08** | **6,34E+07** |
| t1/2 B (min) | 264,4 | 136,8 | 295,6 | 69,7 | 52,5 | **163,8** | **111,2** |
| t1/2 A (min) | 23,9 | 7,4 | 11,0 | 7 | 5,2 | **10,9** | **7,5** |
| Vd(ml/kg) | 1221 | 796 | 1450 | 824 | 707 | **1,00E+03** | **3,20E+02** |
| AUC₀₋₃₀₀ₘᵢₙ-DPM*min | 1,16E+08 | 1.12E+08 | 9,75E+07 | 5,17E+07 | 4,11E+07 | **8,36E+07** | **3,48E+07** |

| Concentration urinaire (DPM/ml) | | | | | | | |
|---|---|---|---|---|---|---|---|
| 0-2h | 1,63E+07 | 4,44E+07 | NS | NS | 1,55E+07 | | |
| 2-5h | 1,51E+07 | 1,38E+07 | 3,26E+07 | NS | 9,66E+06 | | |
| 5-7h | 1,22E+07 | 8,00E+05 | 1,05E+07 | NS | NS | | |
| 7-24h | 1,44E+05 | 2,24E+05 | 5,00E+05 | 6,78E+06 | 4,12E+05 | | |
| 24-30h | 3,54E+05 | 1,56E+05 | 1,92E+05 | 9,42E+05 | 5,08E+04 | | |
| 30-48h | 1,13E+05 | 1,02E+05 | 1,12E+05 | 1.80E+04 | 2,25E+04 | | |
| 48-72h | 6,94E+04 | 7,03E+04 | 8,60E+04 | NS | NS | | |

| Volume urinaire (ml) | | | | | | | |
|---|---|---|---|---|---|---|---|
| 0-2h | 0,96 | 2,61 | NS | NS | 3,24 | | |
| 2-5h | 7,54 | 1,58 | 4,59 | NS | 4,49 | | |
| 5-7h | 2,84 | 2,27 | 1,23 | NS | NS | | |
| 7-24h | 40,08 | 44,14 | 30,53 | 16,3 | 24,03 | | |
| 24-30h | 5,27 | 8,61 | 5,32 | 5,99 | 14,76 | | |
| 30-48h | 41,58 | 41,16 | 31,8 | 19,06 | 48,87 | | |
| 48-72h | 61,46 | 58,63 | 46,55 | NS | NS | | |

| Q urinaire (DPM) | | | | | | | |
|---|---|---|---|---|---|---|---|
| 0-2h | 1.56E+07 | 1,16E+08 | 0.00E+00 | O,OOE+00 | 5,03E+07 | | |
| 2-5h | 1,14E+08 | 2,18E+07 | 1,50E+08 | 0,00E+00 | 4,34E+07 | | |
| 5-7h | 3.45E+07 | 1,81E+06 | 1,28E+07 | 0,00E+00 | 0,00E+00 | | |
| 7-24h | 5,78E+06 | 9,87E+06 | 1,53E+07 | 1.11E+08 | 9,89E+06 | | |
| 24-30h | 1,87E+06 | 1,35E+06 | 1,02E+06 | 5,64E+06 | 7,50E+05 | | |
| 30-48h | 4,69E+06 | 4,22E+06 | 3,56E+06 | 3,43E+05 | 1.10E+06 | | |
| 48-72h | 4,26E+06 | 4,12E+06 | 4,00E+06 | 0,00E+00 | 0,00E+00 | | |
| Total(DPM) | 1,81E+08 | 1,59E+08 | 1.86E+08 | 1,17E+08 | 1,05E+08 | | |
| Q administrée (DPM) | 1,77E+08 | 1,90E+08 | 1,96E+08 | 1,49E+08 | 1,27E+08 | | |
| poids (kg) | 0,3162 | 0,3196 | 0,3507 | 0,3363 | 0,3221 | **0,33** | **0,01** |
| dose (mg/kg) | 0,93 | 1,04 | 1,05 | 1,12 | 0,87 | **1,00** | **0.1** |
| % excrété | 102,0 | 83,9 | 94,9 | 78,4 | 83 | **88,44** | **9,70** |
| CL_{R(0inf)} (ml/min/kg) | 3,3 | 3,4 | 3,2 | 6,4 | 7,8 | **4,81** | **2,13** |
| CLR₍₀₋₃₀₀ₘᵢₙ₎ (ml/min/kg) | 4,9 | 4,5 | 5,4 | 6,7 | 8 | **5,9** | **1,42** |
| Ctotale (ml/min/kg)dose/AUC | 3,2 | 4 | 3,4 | 8,2 | 9.4 | **5,64** | **2,91** |

| | | | | | | | |
|---|---|---|---|---|---|---|---|
| NS : pas d'échantillon | | | | | | | |

**Tableau VI - Groupe n°6 - Inuline-³NH avec Captopril Concentration plasmatique, urinaire et paramètres pharmacocinétiques pour l'Inuline-³H en présence de captopril**

| **Concentration plasmatique (DPM/ml)** | | | | | | | |
|---|---|---|---|---|---|---|---|
| | **rats** | | | | | | |
| temps (min) | 1 | 2 | 3 | 4 | 5 | **moyenne** | **SD** |
| 5 | 1,64E+06 | 2,14E+06 | 8,72E+05 | 1,26E+06 | 5,51E+05 | **1,29E+06** | **6,26E+05** |
| 10 | 9,19E+05 | 1,64E+06 | 6,67E+05 | 7,82E+05 | 6,47E+05 | **9,32E+05** | **4,12E+05** |
| 15 | 7,16E+05 | 1,48E+06 | 5,84E+05 | 6,32E+05 | 6,15E+05 | **8,06E+05** | **3,82E+05** |
| 30 | 3,94E+05 | 1,15E+06 | 4,20E+05 | 3,62E+05 | 6,93E+05 | **6,04E+05** | **3,34E+05** |
| 60 | 1,64E+05 | 7,41 E+05 | 2,52E+05 | 1,58E+05 | 5,02E+05 | **3,63E+05** | **2,53E+05** |
| 120 | 4,97E+04 | 3,67E+05 | 1,08E+05 | 4,60E+04 | 1,89E+05 | **1,52E+05** | **1,33E+05** |
| 180 | 4,21 E+04 | 1,94E+05 | 6,79E+04 | 2,61 E+04 | 6,34E+04 | **7,87E+04** | **6,66E+04** |
| 240 | 2,20E+04 | 1,09E+05 | 2,18E+04 | 2,06E+04 | 4,46E+04 | **4,36E+04** | **3,80E+04** |
| 300 | 2,53E+04 | 2,95E+04 | 2,24E+04 | 1,66E+04 | 4,25E+04 | **2,73E+04** | **9,71E+03** |
| AUC_{0-inf}-DPM/ml*min | 5,25E+07 | 1,47E+08 | 5,16E+07 | 4,38E+07 | 8,40E+07 | **7,57E+07** | **4,26E+07** |
| t1/2 B (min) | 69,9 | 54,4 | 60,6 | 62,7 | 207,8 | **91,1** | **65,5** |
| T1/2 A (min) | 6,4 | 2,2 | 7,7 | 7,1 | 54,9 | **15,7** | **22** |
| Vd(ml/kg) | 952 | 283 | 883 | 886 | 1732 | **9,47E+02** | **5,16E+02** |
| AUC₀₋₃₀₀ₘᵢₙ-DPM*min | 4,99E+07 | 1,45E+08 | 4,96E+07 | 4,23E+07 | 7,05E+07 | **7,14E+07** | **4,22E+07** |

| Concentration urinaire (DPM/ml) | | | | | | | |
|---|---|---|---|---|---|---|---|
| 0-2h | 7,81 E+07 | NS | 3,77E+07 | NS | NS | | |
| 2-4h | 8,16E+06 | NS | 1,92E+07 | NS | NS | | |
| 4-24h | 6,83E+04 | 3,66E+06 | 4,48E+04 | 1,72E+06 | 3,76E+07 | | |

| Volume urinaire (ml) | | | | | | | |
|---|---|---|---|---|---|---|---|
| 0-2h | 1,50 | NS | 1,75 | NS | NS | | |
| 2-4h | 2,7 | NS | 2,75 | NS | NS | | |
| 4-24h | 19,50 | 39,00 | 52,00 | 52,00 | 3,00 | | |

| Q urinaire (DPM) | | | | | | | |
|---|---|---|---|---|---|---|---|
| 0-2h | 1,17E+08 | 0,00E+00 | 6,60E+07 | 0,00E+00 | 0,00E+00 | | |
| 2-4h | 2,20E+07 | 0,00E+00 | 5,28E+07 | 0,00E+00 | 0,00E+00 | | |
| 4-24h | 1,33E+06 | 1,43E+08 | 2,33E+06 | 8,93E+07 | 1,13E+08 | | |
| total(DPM) | 1,40E+08 | 1,43E+08 | 1,21E+08 | 8,93E+07 | 1,13E+08 | | |
| Q administrée (DPM) | 1,50E+08 | 1,60E+08 | 1,58E+08 | 1,37E+08 | 1,51E+08 | | |
| poids (kg) | 0,3030 | 0,3016 | 0,3043 | 0,3185 | 0,3111 | **0,31** | **0,01** |
| dose (mg/kg) | 1,12 | 1,19 | 1,17 | 0,97 | 1,09 | **1,11** | **0,09** |
| % excrété | 93,5 | 89,3 | 76,4 | 65,3 | 74,7 | **79,84** | **11,46** |
| CL_{R*0-inf} (ml/min/kg) | 8,8 | 3,2 | 7,7 | 6,4 | 4,3 | **6,10** | **2,33** |
| CL_{R(0-300min)} (ml/min/kg) | 9,3 | 3,3 | 8 | 6,6 | 5,1 | **6,47** | **2,36** |
| Ctotale (ml/min/kg) | 9,5 | 3,6 | 10,1 | 9,8 | 5,8 | **7,75** | **2,9** |

| | | | | | | | |
|---|---|---|---|---|---|---|---|
| NS : pas d'échantillon | | | | | | | |

**Tableau VII - Tableau Récapitulatif**

| | **AcSDKP sans captopril** | **AcSDKP avec captopril** | **AcSDKP - NH**₂ **sans captopril** | **AcSDKP-NH**₂ **avec captopril** | **Inuline-**³**H sans captopril** | **Inuline-**³**H avec captopril** |
|---|---|---|---|---|---|---|
| % excrété | 10 | 99 | 93 | 99 | 88 | 80 |
| T 1/2 vie (min) | 6,8 | 30,7 | 23,3 | 23,1 | 10,9 (α) | 15,7 (α) |
| | | | | | 163,8 (β) | 91,1 (β) |
| Clairance Rénale (ml/min/kg) | 11,2 | 11,7 | 7,6 | 8,0 | 5,9 | 6,47 |
| Clairance Totale (ml/min/kg) | 144,4 | 11,9 | 8,1 | 8,1 | 5,6 | 7,7 |

### IV Conclusions

D'après les données de la littérature, la clairance rénale chez le rat est comprise entre 5 et 10 ml/min/kg (Woods, 1998 : 10 ml/min/kg - Lin, 1995 : 8,07 ml/min/kg - Davies, 1993 : 5,2 ml/min/kg).

Les pourcentages moyens d'inuline-³H retrouvés dans les urines pour les groupes témoins sont de 88,44 % (sans captopril) et de 79,84 % (avec captopril). Ces valeurs démontrent que (aux erreurs de mesure près), l'inuline-³H est totalement excrétée et que son élimination n'a pas été modifiée par l'administration de captopril. Les clairances rénales moyennes sont de 5,90 ml/min/kg (sans captopril) et de 6,47 ml/min/kg (avec captopril). Les groupes témoins valident le système utilisé (système animal).

Les pourcentages moyens d'AcSDKP retrouvés dans les urines pour les groupes n°1 et n°2 sont de 7,86 % (sans captopril) et de 97% (avec captopril). Ces valeurs démontrent que l'AcSDKP est protégé par l'inhibiteur de l'enzyme de conversion (le captopril) et qu'en absence de captopril, l'AcSDKP est rapidement dégradé *in vivo.* Les clairances rénales moyennes sont de 11,21 ml/min/kg (sans captopril) et de 11,67 ml/min/kg (avec captopril).

Les pourcentages moyens d'AcSDKP-NH₂ retrouvés dans les urines pour les groupes n°3 et n°4 sont de 92,67 % (sans captopril) et de 98,76 % (avec captopril). Ils ne sont donc pas modifiés par l'administration de captopril. La moyenne de l'air sous la courbe (AUC) d'AcSDKP-NH₂ pour le groupe traité sans captopril est inférieure d'environ 10% par rapport à la moyenne de la concentration plasmatique du groupe traité avec le captopril (il faut noter que la dose injectée moyenne pour le groupe traité en présence de captopril est de 11% supérieure au groupe traité sans captopril). Les clairances rénales moyennes sont de 7,59 ml/min/kg (sans captopril) et de 8,03 ml/min/kg (avec captopril). Le temps moyen de ½ vie du peptide est de 23,3 minutes pour le groupe traité sans captopril et de 23,1 minutes pour le groupe traité avec le captopril.

Au vu de ces résultats, on peut conclure que l'AcSDKP-NH₂ n'est dégradé *in vivo* chez le rat ni par l'ECA ni par d'autres systèmes enzymatiques et qu'il est totalement excrété par voie urinaire sans être réabsorbé ni secrété. La courbe de décroissance en fonction du temps, la demi-vie d'élimination, et la clairance urinaire d'AcSDKP-NH₂ ne sont pas influencés par la co-administration de captopril confirmant que le peptide n'est pas dégradé par l'ECA *in vivo.*

Le DFG mesuré après administration d'inuline-[³H] ou d'AcSDKP-NH₂ ne diffère pas significativement entre les différents groupes de rats.

### EXEMPLE 2 : Etude comparative de la dégradation des deux peptides in vitro en présence d'enzyme de conversion animale et humaine.

Les peptides (100 nM) ont été incubés avec un broyat de poumon de rat (à 0,1 mg/ml) ou d'enzyme de conversion humaine (5 mU final) en présence ou non de captopril (10 µM final) dans du tampon Tris/HCl 0,1 M + 0,3 mM de NaCl pH=7,5. Un témoin comportant uniquement les peptides en présence de tampon Tris/HCl 0,1 M + 0,3 mM de NaCl pH=7,5 a été réalisé.

Les incubations ont été : T_{0 min}-T_{30 min}-T_{60 min}-T_{120 min}-T_{240 min} (un temps T_{24 heures} a été réalisé pour l'AcSDKP-NH₂).

Pour arrêter la réaction, une extraction au méthanol a été réalisée. A chaque temps, la concentration du peptide non dégradé a été mesurée par enzymo-immunodosage.

Les résultats sont exprimés en pourcentage de peptide dosé par rapport au témoin pour chaque temps de prélèvement.

**Tableau VIII: Résultats de la dégradation in vitro de l'AcSDKP et de l'AcSDKP-NH₂ en présence d'ACE de poumon de rat.**

| **Temps incubation (min)** | **AcSDKP** | **AcSDKP + captopril** | **AcSDKP-NH**_{**2**} | **AcSDKP-NH**_{**2**} **+ captopril** |
|---|---|---|---|---|
| T_{30 min} (1/1) | 28% | 129% | 100 % | 125% |
| T_{60 min} (1/1) | 9% | 111% | 65% | 65% |
| T_{120 min} (1/1) | 5% | 75% | 95% | 109% |
| T_{240 min} (1/1) | 5% | 89% | 126 % | 136 % |

**Tableau IX : Résultats de la dégradation in vitro de l'AcSDKP et de l'AcSDKP-NH₂ en présence d'ACE humaine.**

| **Temps incubation (min)** | **AcSDKP** | **AcSDKP+ captopril** | **AcSDKP-NH**_{**2**} | **AcSDKP-NH**_{**2**} **+ captopril** |
|---|---|---|---|---|
| T₁₀ₘᵢₙ (1/1) | 82 % | 90 % | 79% | 90 % |
| T₃₀ₘᵢₙ (1/1) | 75% | 100% | 100% | 162% |
| T₆₀ₘᵢₙ (1/1) | 50% | 85% | 116% | 78% |
| T₁₂₀ₘᵢₙ(1/1) | 25% | 85% | 116% | 95% |
| T₂₄₀ₘᵢₙ (1/1) | 7% | 100 % | 107% | 104% |
| T₁₄₄₀ min (1/1) | - | - | 86 % | 117% |

| | | | | |
|---|---|---|---|---|
| - non réalisé. | | | | |

Ces résultats montrent que l'AcSDKP est dégradé par l'ECA de rat ou d'origine humaine et que cette dégradation est inhibée en présence de captopril.

A l'inverse, l'AcSDKP-NH₂ ne semble pas dégradé par l'enzyme de conversion de rat ou d'origine humaine. En effet, les concentrations retrouvées sont de 100 ± 25 %, c'est-à-dire dans les limites de variabilités du dosage.

## Revendications

1. Utilisation des analogues du N-acétyl-Ser-Asp-Lys-Pro, résistants à l'ECA, sélectionnés dans le groupe constitué par les pseudopeptides, dans lesquels l'une des liaisons peptidiques a été remplacée par un groupe amino-méthylène Ψ(CH₂-NH) et un peptide modifié à son extrémité C-terminale par amidation, pour la préparation d'un réactif ou traceur adapté à la mesure du débit de filtration glomérulaire.

2. Utilisation selon la revendication 1, **caractérisée en ce que** lesdits analogues présentent les formules suivantes :
N-acétyl-Ser (CH₂-NH)-Asp-Lys-Pro: SΨ
N-acétyl-Ser-Asp (CH₂-NH)-Lys-Pro: DΨ
N-acétyl-Ser-Asp-Lys(CH₂-NH)-Pro : KΨ et
N-acétyl-Ser-Asp-Lys-Pro-NH₂.

3. Utilisation selon la revendication 1 ou la revendication 2, **caractérisée en ce que** ledit analogue est radiomarqué.

4. Kit ou coffret de mesure du débit de filtration glomérulaire, **caractérisé en ce qu'**il comprend un analogue du N-acétyl-Ser-Asp-Lys-Pro sélectionné dans le groupe constitué par le N-acétyl-Ser (CH₂-NH)-Asp-Lys-Pro (SΨ), le N-acétyl-Ser-Asp (CH₂-NH)-Lys-Pro (DΨ), le N-acétyl-Ser-Asp-Lys(CH₂-NH)-Pro (KΨ) et le N-acétyl-Ser-Asp-Lys-Pro-NH₂ et un moyen de détection dudit analogue dans au moins un échantillon biologique.

5. Kit ou coffret selon la revendication 4, **caractérisé en ce que** le moyen de détection dudit analogue est sélectionné dans le groupe constitué par les immunodosages, les techniques de chromatographie et/ou de spectrométrie de masse.

6. Kit ou coffret selon la revendication 4, **caractérisé en ce que** ledit analogue est radiomarqué et ledit moyen de détection est sélectionné dans le groupe constitué par les techniques d'imagerie et les techniques de comptage radioactif.

## Claims

1. Use of the ACE-resistant N-acetyl-Ser-Asp-Lys-Pro analogs selected from the group consisting of pseudopeptides, in which one of the peptide bonds has been replaced with an aminomethylene Ψ(CH₂-NH) group, and a peptide modified at its C-terminal end by amidation, for preparing a reagent or tracer suitable for measuring glomerular filtration rate.

2. The use according to claim 1, **characterized in that** said analogs have the following formulae:
N-acetyl-Ser(CH₂-NH)-Asp-Lys-Pro: SΨ
N-acetyl-Ser-Asp(CH₂-NH)-Lys-Pro: DΨ
N-acetyl-Ser-Asp-Lys(CH₂-NH)-Pro: KΨ and
N-acetyl-Ser-Asp-Lys-Pro-NH₂.

3. The use according to claim 1 or claim 2, **characterized in that** said analog is radiolabeled.

4. Kit or pack for measuring glomerular filtration rate, **characterized in that** it comprises an N-acetyl-Ser-Asp-Lys-Pro analog selected from the group consisting of N-acetyl-Ser(CH₂-NH)-Asp-Lys-Pro (SΨ), N-acetyl-Ser-Asp (CH₂-NH) -Lys-Pro (DΨ), N-acetyl-Ser-Asp-Lys(CH₂-NH)-Pro (KΨ) and N-acetyl-Ser-Asp-Lys-Pro-NH₂, and a means of detecting said analog in at least one biological sample.

5. The kit or pack according to claim 4, **characterized in that** the means of detecting said analog is selected from the group consisting of immunoassays, chromatography techniques and/or mass spectrometry techniques.

6. The kit or pack according to claim 4, **characterized in that** said analog is radiolabeled and said detection means is selected from the group consisting of imaging techniques and radioactive counting techniques.

## Patentansprüche

1. Verwendung der Analoga des N-Acetyl-Ser-Asp-Lys-Pro, die gegenüber dem ACE resistent sind, ausgewählt aus der Gruppe, bestehend aus den Pseudopeptiden, wobei eine der peptidischen Bindungen durch eine Aminomethylengruppe Ψ(CH₂-NH) ersetzt und ein Peptid an seinem C-terminalen Ende durch Amidierung modifiziert wurde, zur Herstellung eines Reagens oder eines Tracers, das/der zur Messung des Durchsatzes der glomerulären Filtration geeignet ist.

2. Verwendung nach Anspruch 1, **dadurch gekennzeichnet, dass** die Analoga die folgenden Formeln aufweisen:
N-Acetyl-Ser (CH₂-NH)-Asp-Lys-Pro: Sψ
N-Acetyl-Ser-Asp (CH₂-NH)-Lys-Pro: Dψ
N-Acetyl-Ser-Asp-Lys(CH₂-NH)-Pro: Kψ und
N-Acetyl-Ser-Asp-Lys-Pro-NH₂.

3. Verwendung nach Anspruch 1 oder Anspruch 2, **dadurch gekennzeichnet, dass** das Analogon radioaktiv markiert ist.

4. Kit oder Testsatz zur Messung des Durchsatzes der glomerulären Filtration, **dadurch gekennzeichnet, dass** es ein Analogon von N-Acetyl-Ser-Asp-Lys-Pro, ausgewählt aus der Gruppe, bestehend aus N-Acetyl-Ser (CH₂-NH)-Asp-Lys-Pro (Sψ), N-Acetyl-Ser-Asp (CH₂-NH)-Lys-Pro (Dψ), N-Acetyl-Ser-Asp-Lys(CH₂-NH)-Pro (Kψ) und N-Acetyl-Ser-Asp-Lys-Pro-NH₂, und ein Mittel zum Nachweis des Analogons in mindestens einer biologischen Probe umfasst.

5. Kit oder Testsatz nach Anspruch 4, **dadurch gekennzeichnet, dass** das Nachweismittel des Analogons aus der Gruppe ausgewählt ist, bestehend aus immunanalytischen Techniken, chromatographischen Techniken und/oder Massenspektrometrie.

6. Kit oder Testsatz nach Anspruch 4, **dadurch gekennzeichnet, dass** das Analogon radioaktiv markiert ist und das Nachweismittel aus der Gruppe ausgewählt ist, bestehend aus Abbildungstechniken und radioaktiven Zähltechniken.
